# EUROPEAN PATENT APPLICATION

(11) **EP 2 762 572 A1**
(43) Date of publication of application: **06.08.2014**
(21) Application number: 13154102.1
(22) Date of filing: 05.02.2013
(51) Int. Cl.: C12Q 1/68

(54) **In vitro method for determining skin sensitizing potential.**

(71) Applicant: Universiteit Maastricht, 6211 LK Maastricht (NL); Academisch Ziekenhuis Maastricht, 6229 HX Maastricht (NL); Rijksinstituut Voor Volksgezondheid En Milieu, 3721 MA Bilthoven (NL)
(72) Inventor: Van Loveren, Hendrik, 3734 CN Den Dolder (NL); Van der Veen, Jochem Willem, 6200 MD Maastricht (NL); Goossens, Hendrika Maria, 3720 BA Bilthoven (NL); Pennings, Jeroen Lambertus Antonius, 3720 BA Bilthoven (NL); Pronk, Theresia Elizabeth, 3994 XR Houten (NL); Dollé, Martijn Emile Theodoor, 3720 BA Bilthoven (NL)
(74) Representative: Habets, Winand

(57) **Abstract**

The invention is in the field of in vitro test methods for the determination of properties of certain test compounds. More in particular, the invention provides a method for the determination of the skin sensitizing properties of a compound. For that purpose, the expression level of certain microRNAs is determined. More specifically, the invention provides a method for determining whether a test compound has skin sensitizing potential by exposing a keratinocyte cell in a cell culture to the test compound with, determining the level of expression of microRNA 1973 and concluding that the compound has skin sensitizing potential when the expression level of microRNA 1973 is above a predetermined reference value.

## Description

### Field of the invention

The invention is in the field of in vitro test methods for the determination of properties of certain test compounds. More in particular, the invention provides a method for the determination of the skin sensitizing properties of a compound. For that purpose, the expression level of certain microRNAs is determined.

### Background of the invention

Allergic contact dermatitis is a delayed-type IV hypersensitivity reaction that can be induced after skin contact with chemical haptens. It is a common occupational and consumer health problem which develops through a series of immunological events caused by repeated contact with compounds that have skin sensitizing potential (Kimber et al., 2002). The current methods for assessing the sensitizing potential of chemicals are the Local Lymph Node Assay (LLNA) or guinea pig tests (Guinea Pig Maximization Test (GPMT) or Buehler test) (Gerberick et al., 2007; Kimber et al., 1994). There is great demand for validated non-animal alternatives to replace these animal tests, due to the ban on animal testing described in the 7th amendment to the European Union Cosmetics Directive. In addition, the REACH (Registration, Evaluation, and Authorization of Chemicals) regulation requires that the safety of a large amount of chemicals has to be assessed and it stimulates the use of alternative test methods. These legislative changes, combined with ethical issues and societal acceptance towards animal use in toxicity testing, drive further development of alternative test methods.

Although much progress has been made for assessing skin sensitizing potential, no alternative test methods have been validated yet. In recent years, it has become clear that a combination of methods in a testing strategy will be required for correct identification of sensitizers, rather than a single test (Vandebriel and van Loveren, 2010).

Many of current cell based alternative assays for skin sensitization use either keratinocytes or dendritic cells (van der Veen et al., 2013). Read-outs are either changes in gene regulation in these cells (Arkusz et al., 2010; Johansson et al., 2011; Natsch, 2009; Vandebriel et al., 2010), production of cytokines, such as IL-18 in keratinocytes (Corsini et al., 2009) or upregulation of cell surface markers, including CD86 and CD54 on dendritic cells (Aeby et al., 2004; Ashikaga et al., 2006; Sakaguchi et al., 2006; Schreiner et al., 2008) exposed to sensitizers. The prediction accuracy of these assays range between 71 % and 99% (Bauch et al., 2011).

### Summary of the invention.

We found that microRNA 1973 may be used as a marker for skin sensitizing compounds. The invention provides an in vitro method for determining whether a compound has skin sensitizing potential by contacting the compound with a keratinocyte cell, determining the level of expression of microRNA 1973 and concluding that the compound has skin sensitizing potential when the expression level of microRNA 1973 is above a predetermined reference value.

### Detailed description of the invention.

We observed that keratinocyte cells react to exposure with skin sensitizers with an increased expression of certain microRNAS, in particular hsa-miR-1973, also referred to as miRNA 1973 or microRNA 1973. In this way, compounds with skin sensitizing properties may be identified, thereby obviating the need for exposure of animals to the compound.

The invention therefore provides an in vitro method for determining whether a compound has skin sensitizing potential by contacting the compound with a keratinocyte cell, determining the level of expression of microRNA 1973 and concluding that the compound has skin sensitizing potential when the expression level of microRNA 1973 is above a predetermined reference value.

MicroRNA 1973 is known in the art. It is a short RNA molecule with primary sequence
UAUGUUCAACGGCCAUGGUAUCCUGACCGUGCAAAGGUAGCAUA (SEQ ID NO: 1). It's function is currently unknown, it has been implicated in the etiology of Acute Lymphoblastic Leukemia (Schotte D, et al. Leukemia, 2009 Feb. PMID 18923441).

Detection of microRNAs has also been described. There are a number of ways for determining the expression level of microRNAs, including miRNA 1973. The skilled person is aware of these techniques and their relative advantages and disadvantages. As an example only, the examples section herein describes the detection of miRNA 1973 using a polymerase chain reaction (PCR) assay. Methods employing Northern blotting techniques or array hybridisations, either solid state or liquid based (e.g. Luminex), may be used as well. Methods employing quantitative PCR and nucleic acid sequence based amplification (NASBA) or gene sequencing may be equally well employed.

The expression levels of the miRNA 1973 marker are to be compared to a reference value, also often referred to as cut-off value. Such a reference value may be empirically determined or arbitrarily chosen in order to achieve appropriate specificity and/or sensitivity of the method. A skilled person is fully aware how to choose an appropriate reference value. Such a reference value may advantageously be based on a control expression value.

The control expression value is defined herein as the expression value for a particular gene obtained with a control exposed cell instead of a cell exposed to a sensitizing compound. A skilled person is fully aware of ways to determine a control expression value for a particular gene. This may be done using control cells exposed to identical conditions as the test cells with the exception that the control cell is not exposed to the compound to be tested for sensitizing properties. Control expression values may also be obtained using a standard gene expression in a quantitative PCR assay. Such a standard gene is then advantageously a housekeeping gene.

The control expression value is used to calculate the ratio of the gene expression as detailed herein. A skilled person will know how to alter the predetermined reference value in order to obtain the desired specificity and sensitivity of the method.

In a preferred embodiment, it is concluded that a test compound has skin sensitizing potential when the expression level of the microRNA 1973 is more than 25% above the reference value, in other words, the ratio is at least 1,25.

In a method according to the invention, it is preferred to contact the keratinocyte cell with the test compound in a culture medium.

We also found that a number of other microRNAs were suitable as markers for skin senzitation in addition to the microRNA 1973 marker. We performed an experiment wherein the RNA samples of the cells exposed to the sensitizing compounds in table 1 were pooled. The samples exposed to the irritant components and the controls were also pooled. The pooled samples showed significant (p < 0,05) differential expression of 5 other microRNAs. MicroRNA 300 and 29b-1* were overexpressed whereas microRNA 192, 3607-5p and 600 were underexpressed.

| **Probe ID** | **microRNA** | **Differential expression [ratio]** | **P value** |
|---|---|---|---|
| 42513 | hsa-miR-300 | 1,86 | 2.06E-03 |
| 17732 | hsa-miR-192 | -0,56 | 9.56E-03 |
| 148418 | hsa-miR-3607-5p | -0,70 | 1.47E-02 |
| 17377 | hsa-miR-600 | -0,73 | 2.07E-02 |
| 17810 | hsa-miR-29b-1* | 1,78 | 3.23E-02 |

The invention therefore also relates to a method as described above wherein in addition to the step of determining the level of expression of microRNA 1973, also the level of expression of at least one microRNA is determined selected from the group consisting of microRNAs 300, 29b-1*, 192, 3607-5p and 600.

It appeared that the method works particularly well when the mean expression value of microRNA 1973 in keratinocyte cells exposed to at least one control compound is taken as the reference value. The invention therefore relates to a method as described above wherein the reference level is the mean expression value of microRNA 1973 in keratinocyte cells exposed to at least one control compound.

The method according to the invention may also be used to distinguish compounds with a skin sensitizing potential from irritants. The reference value may be derived from a mean value of expression measured in cells exposed to at least one irritant.

We found that the use of a keratinocyte cell line termed HaCaT was particularly useful. The invention therefore relates to a method as described above, wherein the keratinocyte cell is a HaCaT cell.

### Legend to the figures

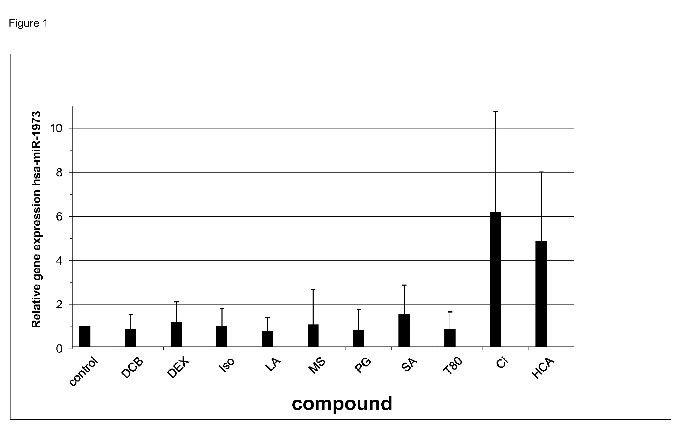

- Figure 1:: Graph showing the relative expression of microRNA 1973 in HaCaT cells exposed to different compounds as indicated. Control is cells exposed to the vehicle (ethanol).

### Examples

### Example 1: Cell culture.

The human keratinocyte cell line HaCaT (Boukamp et al., 1988) was purchased from Cell Lines Service (Heidelberg, Germany). Cells were grown in culture flasks to 80% confluence in Dulbecco's modified Eagle's medium (DMEM), supplemented with 1% nonessential amino acids, 100 U/ml penicillin, 100 µg/ml streptomycin (all from Gibco, Breda, the Netherlands), and 10% heat-inactivated Fetal Calf Serum (Integro, Zaandam, the Netherlands) (complete medium), at 37 degrees C in a humidified atmosphere of 5% CO2 in air. For passaging the cells were washed twice with PBS and then trypsinized (0.05% Trypsin with EDTA 4Na; Gibco). New culture flasks were seeded in complete medium with 1/3rd or 1/6th of the total number of cells of the previous passage.

### Example 2: Exposure of cells

Before exposure, trypsinized cells were resuspended in fresh complete medium to a concentration of 3 x 10E5 cells/ml. The cell suspension was seeded into 12-well plates (1.5 ml per well; Greiner, Alphen aan den Rijn, the Netherlands). The cells were allowed to adhere and form a monolayer during 24 h, after which the wells were washed with PBS and exposed to the different test compounds in complete medium. The compounds that were used in this study are shown in Table 1.

**Table 1; Compounds used in the study.**

| **Compound** | **Abbr.** | **CAS #** | **Irritant/ Sensitizer** | **Concentration** |
|---|---|---|---|---|
| Chlorobenzene | DCB | 108-90-7 | Irritant | 21.88 µL / mL |
| Dextran | DEX | | Irritant | 2.00 mg / mL |
| 2-Propanol | ISO | 67-63-0 | Irritant | 1.91 µL / mL |
| Lactic Acid | LA | 50-21-5 | Irritant | 0.3 µL / mL |
| Methyl salicylate | MS | 119-36-8 | Irritant | 36.36 µL / mL |
| Propylene Glycol | PG | | Irritant | 29.53 µL / mL |
| Salicylic acid | SA | 69-72-7 | Irritant | 34.67 mg / mL |
| Tween-80 | T80 | | Irritant | 1 µL/mL |
| Citral | Ci | 5392-40-5 | Sensitizer | 3.89 µL / mL |
| Alpha-hexylcinnamaldehyde | HCA | 101-86-0 | Sensitizer | 3.30 µL/mL |

All compounds were obtained from Sigma-Aldrich (Zwijndrecht, the Netherlands. Compounds were dissolved in absolute ethanol. In addition to the compound, the vehicle (ethanol) was added to a final concentration of 1% to the cell cultures exposed to compounds dissolved in complete medium. Cells were exposed to ethanol to obtain vehicle control samples. For each compound the concentration resulting in 80% viability (CV80) was determined using colorimetric measurement of WST-1 cleavage.

To this end, HaCaT cells were seeded in 12-well plates (4.5 x 10E5 cells/well) and incubated with a concentration range of the compounds in duplicate or solvent control in triplicate at 37 degrees C in a humidified atmosphere of 5% CO2 in air. After 21 h of exposure, 1.2 mL medium was removed from the wells, leaving 300 microliter exposure medium in the wells, and 40 microliter/well WST-1 (Roche, Woerden, the Netherlands) was added. After 4 h, 100 microliter was transferred to a microtiter plate and WST-1 cleavage was quantified at 450 nm using a microplate reader (Spectramax 190, Molecular devices, Wokingham, UK). After blank correction, WST-1 without cells, the mean optical densities of the replicates were compared to the mean of the corresponding vehicle controls in order to calculate relative viability.

In a single experiment the HaCaT cells were exposed to each compound in four replicates for 4 h.

### Example 3: RNA isolation

At the end of the exposure time, 400 microliter RNAprotect cell reagent (Qiagen Benelux B.V., Venlo, the Netherlands) was added to each well. Cells were resuspended and were stored at -20 degrees Celsius until further analysis. RNA was isolated by using the miRNeasy Mini Kit by Qiagen according to the manufacturer's instructions. RNAprotect was removed and cells were lysed in Qiazol by mixing thoroughly.

RNA quantity was spectrophotometrically assessed (Nanodrop Technologies, Wilmington, DE), and integrity was determined by automated gel electrophoresis (Bioanalyzer 2000; Agilent technologies, Amstelveen, the Netherlands)

### Example 4: Determination of expression of miRNA 1973

Expression levels of miR-1973 were measured by performing quantitative-PCR expression analysis on isolated RNA of the individual exposures. All reagents and equipment were obtained from Applied Biosystems and all reactions were performed according to protocol "Run 96- or 384-well plates without preamplification" as described in user bulletin "Publication part number 4465407, revision date 30 March 2012 rev. B". The user bulletin describes how to pool up the reverse transcriptase (RT) primers for multiplexing the RT step, the performance was validated by comparing values of threshold cycle (Ct) with the individual protocol. The TaqMan microRNA assay hsa-miR-1973 (assay ID 245468_mat) was used and as endogenous controls RNU44 (assay name 568907, assay ID 001094) and hsa-miR-320a (assay ID 002277) were used. In brief 667 ng of total RNA was reversed-transcribed in a total volume of 10 µl using the TaqMan MicroRNA RT kit (part no. 4366596). The RT product was 31x diluted, 5 µl was mixed with 1 µl TaqMan micro RNA assay, 4 µl nuclease-free water and 10 µl TaqMan universal master mix II (part no. 4440040) and was amplified using the 7500 Fast Real-Time PCR System. Ct values were derived from the amplification plots constructed of the ROX-normalized fluorescence signals by SDS Software v2.0.5. The expression levels of the endogenous controls were comparable to each other and for all samples. The mean of the RNU44 and miRNA-320a levels of all samples were therefore used to normalize the expression of miRNA-1973. Relative quantification was performed by the comparative CT method (ddCt) using Microsoft Excel.

The results are shown in table 2 and figure 1.

**Table 2: Relative expression of hsa-miR-1973**

| **Relative gene expression of hsa-miR-1973 vs control** | | **control** |
|---|---|---|
| EtOH | control | 1,000 |
| Chlorobenzene | DCB | 0,874 |
| Dextran | DEX | 1,192 |
| 2-Propanol | ISO | 1,006 |
| Lactic acid | LA | 0,785 |
| Methyl salicylate | MS | 1,082 |
| Propylene glycol | PG | 0,840 |
| Salicyclic acid | SA | 1,559 |
| Tween80 | T80 | 0,867 |
| Citral | Ci | 6,179 |
| α-Hexylcinnamaldehyde | HCA | 4,879 |

SEQUENCE LISTING

<110>  RIVM

<120>  IN VITRO METHOD FOR DETERMINING SKIN SENSITIZING POTENTIAL

<130>  245 EPP0

<160>  1

<170>  PatentIn version 3.5

<210>  1
<211>  44
<212>  RNA
<213>  homo sapiens

<400>  1
uauguucaac ggccauggua uccugaccgu gcaaagguag caua
44

### References

Arkusz, J., Stepnik, M., Sobala, W., Dastych, J., 2010. Prediction of the contact sensitizing potential of chemicals using analysis of gene expression changes in human THP-1 monocytes. Toxicol. Lett. 199, 51-59.
Sakaguchi, H., Ashikaga, T., Miyazawa, M., Yoshida, Y., Ito, Y., Yoneyama, K., Hirota, M., Itagaki, H., Toyoda, H., Suzuki, H., 2006. Development of an in vitro skin sensitization test using human cell lines; human Cell Line Activation Test (h-CLAT). II. An inter-laboratory study of the h-CLAT. Toxicol. In Vitro 20, 774-784.
Aeby, P., Wyss, C., Beck, H., Griem, P., Scheffler, H., Goebel, C., 2004. Characterization of the sensitizing potential of chemicals by in vitro analysis of dendritic cell activation and skin penetration. J. Invest. Dermatol. 122, 1154-1164.
Ashikaga, T., Yoshida, Y., Hirota, M., Yoneyama, K., Itagaki, H., Sakaguchi, H., Miyazawa, M., Ito, Y., Suzuki, H., Toyoda, H., 2006. Development of an in vitro skin sensitization test using human cell lines: the human Cell Line Activation Test (h-CLAT). I. Optimization of the h-CLAT protocol. Toxicol. In Vitro 20, 767-773
Schreiner, M., Peiser, M., Briechle, D., Stahlmann, R., Zuberbier, T., Wanner, R., 2008. A new dendritic cell type suitable as sentinel of contact allergens. Toxicology 249, 146-152.
Johansson, H., Lindstedt, M., Albrekt, A.S., Borrebaeck, C.A., 2011. A genomic biomarker signature can predict skin sensitizers using a cell-based in vitro alternative to animal tests. BMC Genomics 12, 399.
Natsch, A., 2009. The Nrf2-Keap1-ARE toxicity pathway as a cellular sensor for skin sensitizers-functional relevance and a hypothesis on innate reactions to skin sensitizers. Toxicol. Sci. 23, 23-56.
Vandebriel, R.J., van Loveren, H., 2010. Non-animal sensitization testing: state-of the-art. Crit. Rev. Toxicol. 40, 389-404.
Kimber, I., Basketter, D.A., Gerberick, G.F., Dearman, R.J., 2002. Allergic contact dermatitis. Int. Immunopharmacol. 2, 201-211
Kimber, I., Dearman, R.J., Scholes, E.W., Basketter, D.A., 1994. The local lymph node assay: developments and applications. Toxicology 93, 13-31.
Gerberick, G.F., Ryan, C.A., Dearman, R.J., Kimber, I., 2007. Local lymph node assay (LLNA) for detection of sensitization capacity of chemicals. Methods 41, 54-60.
Vandebriel, R.J., Pennings, J.L., Baken, K.A., Pronk, T.E., Boorsma, A., Gottschalk, R., Van Loveren, H., 2010. Keratinocyte gene expression profiles discriminate sensitizing and irritating compounds. Toxicol. Sci. 117, 81-89.
Corsini, E., Mitjans, M., Galbiati, V., Lucchi, L., Galli, C.L., Marinovich, M., 2009. Use of IL-18 production in a human keratinocyte cell line to discriminate contact sensitizers from irritants and low molecular weight respiratory allergens. Toxicol. In Vitro 23, 789-796.
Bauch, C., Kolle, S.N., Fabian, E., Pachel, C., Ramirez, T., Wiench, B., Wruck, C.J., Ravenzwaay, B.V., Landsiedel, R., 2011. Intralaboratory validation of four in vitro assays for the prediction of the skin sensitizing potential of chemicals. Toxicol. In Vitro 10, 567-734.
Van der Veen et al., Toxicology in vitro 27 (2013) 314-322.

SEQUENCE LISTING

<110>  RIVM

<120>  IN VITRO METHOD FOR DETERMINING SKIN SENSITIZING POTENTIAL

<130>  245 EPP0

<160>  1

<170>  PatentIn version 3.5

<210>  1
<211>  44
<212>  RNA
<213>  homo sapiens

<400>  1
uauguucaac ggccauggua uccugaccgu gcaaagguag caua                44

## Claims

1. An *in vitro* method for determining whether a test compound has skin sensitizing potential by exposing a keratinocyte cell in a cell culture to the test compound with, determining the level of expression of microRNA 1973 and concluding that the compound has skin sensitizing potential when the expression level of microRNA 1973 is above a predetermined reference value.

2. Method according to claim 1 wherein in addition to the step of determining the level of expression of microRNA 1973, also the level of expression of at least one microRNA is determined selected from the group consisting of microRNAs 300, 29b-1*, 192, 3607-5p and 600.

3. Method according to claims 1 or 2 wherein the reference level is the mean expression value of microRNA 1973 in keratinocyte cells exposed to at least one control compound.

4. Method according to claim 3 wherein the at least one control compound is an irritant.

5. Method according to any one of claims 3 or 4 wherein the reference value is obtained from at least 2 or 3 or 4 or 5 or 6 or7 or 8 or 9 or 10 or more control compounds.

6. Method according to claims 1 - 5 wherein the keratinocyte cell is a human cell

7. Method according to claim 6 wherein the keratinocyte cell is a HaCaT cell.
